# EUROPEAN PATENT APPLICATION

(11) **EP 3 915 549 A1**
(43) Date of publication of application: **01.12.2021**
(21) Application number: 20176517.9
(22) Date of filing: 26.05.2020
(51) Int. Cl.: A61K 31/047, A61K 31/12, A61K 31/167, A61K 31/22, A61K 31/437, A61K 31/4745, A61K 31/496, A61K 31/519, A61K 31/538, A61K 31/704, A61K 31/706, A61K 31/7072, A61K 33/243, A61K 38/14, A61K 45/06, A61P 31/12, A61P 31/20

(54) **SENESCENCE INDUCERS FOR USE IN THE TREATMENT AND/OR PREVENTION OF VIRUS INDUCED DISEASES**

(71) Applicant: Merck Patent GmbH, 64293 Darmstadt (DE)
(72) Inventor: The designation of the inventor has not yet been filed

(57) **Abstract**

Suggested are one or more compounds for use in the treatment and/or prevention of virus induced diseases, wherein the one or more compound is an inducer of stress-induced premature senescence.

## Description

### Field of the invention

The present invention relates to compounds, in particular inducers of stress-induced premature senescence, for use in the treatment and/or prevention of virus induced diseases.

### Background Art

Cellular senescence is a stable arrest of the cell cycle and is characterized by complex phenotypic changes.
Senescence can be classified as replicative senescence or Stress induced premature senescence (SIPS). Replicative senescence results from a combination of events that includes the progressive erosion of telomeres owing to the accumulation of replication cycles and the increased expression of cyclin-dependent kinase inhibitors (CDKIs). In particular, telomere erosion can result in critically short telomeres that are sensed by the cells as double-strand breaks (DSBs) in DNA, which in turn promotes replicative senescence. These DSBs elicit a DNA damage response (DDR) to transduce signals and to execute senescence, resulting in the formation of γ-H2AX-positive senescence-associated DNA damage foci (SDF) and the activation of the ATM and ATR kinases, which signal downstream to p53. In addition, both INK4A (also known as p16; a CDKI that operates upstream of RB) and the RB tumour suppressor have important roles in replicative senescence.

Stress induced premature senescence can be brought on by a number of different pathways, some of which are the same as those involved in replicative senescence. Stress induced premature senescence can be further grouped into Oncogene induced senescence (OIS), PTEN loss-induced cellular senescence (PICS), and other yet unclassified pathways.

OIS was originally shown to be an in vitro response triggered by cells to prevent oncogenic transformation. In the case of OIS induction that is driven by HRASG12V overexpression in human fibroblasts it has been established that this response is triggered by hyperproliferation and DNA hyper-replication. This in turn sparks the activation of an S phase-specific DNA damage response. Although this DNA damage response is initiated by a mechanism that is distinct from that of replicative senescence, it shares effectors and primary pathways and also results in senescence-associated DNA damage foci. Indeed, OIS fails in cells that lack ATM activity or when cells cannot sense DNA damage or transduce DNA damage response signals to p53.
p53 itself represents an important effector of OIS, through transcriptional activation of target genes, including CDKN1A (encoding the protein p21). In OIS, the activation of p53 is driven by phosphorylation, with concomitant stabilization of the protein mediated by ARF induction. In addition to p53, OIS also engages other senescence effectors, including INK4A (for example, through ETS2) and derepression of the genomic locus through inactivation of the polycomb group complex.
PICS represents a distinct form of senescence that is rapidly induced on Pten loss and that is characterized by the absence of an 'OIS-like' hyper-replication, as well as a lack of a DNA damage response. Indeed, PICS can occur in cells that are treated with aphidicolin, which blocks S phase entry and prevents DNA replication. This is different from HRASG12V-induced OIS, in which aphidicolin treatment results in the abrogation of senescence. Furthermore, the formation of γ-H2AX-positive senescence-associated DNA damage foci is also not observed in PICS, and inhibition of ATM has no effect on PICS induction.
Similar to OIS, p53 has an important role in PICS. However, in this context p53 upregulation is mainly promoted by mTOR-mediated translation. In addition, the genetic inactivation of the gene encoding ARF at the Cdkn2a locus does not dramatically alter p53 levels on the induction of PICS, although it results in a marked reduction of p53 in OIS. Furthermore, genetic deletion of the gene encoding ARF at the Cdkn2a locus in vivo fails to prevent PICS induction in prostate tumorigenesis, demonstrating the dispensable nature of ARF for PICS41.

Several chemical compounds that specifically target senescent cells have been identified in the last 2 years (so-called senolytic drugs) (Xu M, Tchkonia T, Ding H, Ogrodnik M, Lubbers ER, Pirtskhalava T, White TA, Johnson KO, Stout MB, Mezera V, Giorgadze N, Jensen MD, LeBrasseur NK, Kirkland JL (2015b) JAK inhibition alleviates the cellular senescence-associated secretory phenotype and frailty in old age, Proc. Natl Acad. Sci. USA 112, E6301-E6310). It was shown that clearance of senescent cells by such drugs may alleviate age-related vasomotor dysfunction and frailty, enhance adipogenesis, rejuvenate haematopoietic stem cells after total-body irradiation, and, generally, extend lifespan (Xu M, Palmer AK, Ding H, Weivoda MM, Pirtskhalava T, White TA, Sepe A, Johnson KO, Stout MB, Giorgadze N, Jensen MD, LeBrasseur NK, Tchkonia T, Kirkland JL (2015a), Targeting senescent cells enhances adipogenesis and metabolic function in old age, Elife 4, e12997). Furthermore, these studies confirm the known pathological impact of cellular senescence, exemplified by cellular dysfunction, impairment of tissue regeneration, detrimental effects on tissue microenvironment, etc. It is evident that along with its detrimental effects, cellular senescence has clearly defined beneficial physiological functions. For instance, it has been shown recently that cellular senescence plays a role in the differentiation of megakaryocytes, the maturation of the placenta, the restriction of fibrosis, tissue repair and embryonic development. The role of cellular senescence in cancer prevention is also well documented (Burton DG, Krizhanovsky V (2014), Physiological and pathological consequences of cellular senescence, Cell. Mol. Life Sci. 71, 4373-4386).

It is generally agreed in the field that the most important features of cellular senescence are senescence-associated secretory phenotype and resistance to apoptosis. Senescence-associated secretory phenotype stimulates immune system-dependent elimination of unwanted precancerous cells or specific embryonic cells that undergo senescence. Notably, cellular senescence may serve as an alternative to apoptosis in embryonic development as well as in cancer prevention.

However, besides the above described diseases, also virus-induced diseases are a major problem, especially for elder people as well as for people with pre-existing conditions.
Therefore, there is a need to identify compounds that can effectively protect individuals from virus infections and its associated risks.

### Summary of the invention

The aim of the present invention is to provide compounds, in particular inducers of stress-induced premature senescence. The compounds of the present invention are able to induce cellular anti-viral defenses via senescence and therefore to treat and/or prevent individuals from virus infections and their associated diseases.

The present invention therefore relates to one or more compound for use in the treatment and/or prevention of virus induced diseases, wherein the compound is an inducer of stress-induced premature senescence.

Unless defined otherwise, all technical and scientific terms used herein have the same meaning as commonly understood by those of ordinary skill in the art to which this invention belongs. Generally, nomenclatures utilized in connection with, and techniques of, cell and molecular biology and chemistry are those well-known and commonly used in the art. Certain experimental techniques, not specifically defined, are generally performed according to conventional methods well known in the art and as described in various general and more specific references that are cited and discussed throughout the present specification. For purposes of clarity, the following terms are defined below.
The term "one or more compound" refers to the compounds according to the present invention, which are inducers of stress-induced premature senescence. Although the word "compound" is mainly used herein in the singular form, the context makes it clear that the plural form, i.e. several "compounds" shall also be included, except explicitly indicated otherwise. The same also applies when the plural form is used herein.

As explained above, "inducers of stress-induced premature senescence" in the sense of the present invention are compounds, that are able to induce the pathway of stress-induced premature senescence.

The inventors have surprisingly found that compounds that are known inducers of stress-induced premature sensescence can be used for the treatment and/or prevention of virus-induced diseases to their ability to induce cellular anti-viral defenses via senescence.

The inducers of stress-induced premature senescence can be functionally classified into eight groups. In a preferred embodiment according to the invention, the one or more compounds are selected from the group consisting of DNA replication stress inducers, DNA-damaging agents, epigenetic modifiers, inhibitors of telomerase activity, inhibitors of cyclin-dependent kinases, activators of p53, activators of protein kinase C and reactive oxygen species inducers.

"DNA replication stress inducers" are compounds that are able to induce stress during DNA replication. "DNA replication stress" is defined as the events that take place when the genome is exposed to various stresses. It occurs during DNA replication, and can result in a stalled replication fork. "DNA damaging agents" are compounds that are able to cause damage in DNA bases or its sugar phosphate backbone. Furthermore, some DNA damaging agents may cause the formation of covalent bonds between DNA and proteins (DNA protein cross-links) thus affecting DNA-histone and DNA-transcription factor interactions. This has impact on DNA packing (in the case of histones), cell division, replication and transcription of DNA. "Epigenetic modifiers" are compounds that are able to trigger functionally relevant changes to the genome that do not involve a change in the nucleotide sequence. Examples of mechanisms that produce such changes are DNA methylation and histone modification, each of which alters how genes are expressed without altering the underlying DNA sequence. "Inhibitors of telomerase activity" are compounds that are able to inhibit the catalytic activity of enzyme through the targeting of its catalytic components or RNA.
"Inhibitors of cyclin-dependent kinases" are compounds which are able to inhibit the enzyme cyclin-dependent kinase (CDK).
"Activators of p53" are compounds which are able to activate p53 and, in some cases, other cell death and cell differentiation related proteins. "p53" is a DNA-binding tumor suppressor, which upregulates growth arrest and apoptosis-related genes in response to stress signals, thereby influencing programmed cell death, cell differentiation and cell cycle control mechanisms.
"Activators of protein kinase C" are compounds that are able to activate protein kinase C. "Protein kinase C" is a family of protein kinase enzymes that are involved in controlling the function of other proteins through the phosphorylation of hydroxyl groups of serine and threonine amino acid residues on these proteins, or a member of this family.
"Reactive oxygen species inducers" are compounds that are able to induce reactive oxygen species. "Reactive oxygen species" is a group of ions and molecules, including hydroxyl radicals (OH), alkoxyl radicals, superoxide anion (O2-), singlet oxygen (102) and hydrogen peroxide (H2O2).
In a preferred embodiment according to the invention, the DNA replication stress inducers are selected from the group consisting of aphidicolin, hydroxyurea, thymidine, bromodeoxyuridine, difluorodeoxycytidine and cyclopentenyl cytosine.
In a further preferred embodiment according to the invention, the DNA-damaging agents are selected from the group consisting of DNA topoisomerase inhibitors, DNA cross-linkers and drugs with complex effects.
"DNA topoisomerase inhibitors" are compounds that block the action of topoisomerases (topoisomerase I and II), which are enzymes that control the changes in DNA structure by catalyzing the breaking and rejoining of the phosphodiester backbone of DNA strands during the normal cell cycle. "DNA cross-linkers" are compounds which are able to react with two nucleotides of DNA, forming a covalent linkage between them. This crosslink can occur within the same strand (intrastrand) or between opposite strands of double-stranded DNA (interstrand).
"Drugs with complex effects" are compounds that have more than one effect when administered to a person in need thereof. Often, it depends on the dose of the compound which effect occurs. For example, the compound actinomycin D is a drug with complex effect in the sense of the invention. Actinomycin D acts as an intercalator of DNA. Translated into a simple comparison picture, this means that actinomycin D blocks the zipper DNA so that it cannot be opened. Normal use of DNA is not possible without opening. All cells that contain DNA are affected by the effects of actinomycin D. Only the brain cells and spinal cord cells are protected from the effects of actinomycin D by the blood-brain barrier. At low doses, actinomycin D inhibits DNA-dependent RNA synthesis by intercalation (attachment and cross-linking) of the guanine nucleotides in the DNA. At higher doses, actinomycin D also inhibits DNA replication in blocking the activity of the DNA polymerase.

In a preferred embodiment according to the invention, the DNA topoisomerase inhibitors are selected from the group consisting of doxorubicin, etoposide, daunorubicin, mitoxantrone and camptothecin. In a more preferred embodiment according to the invention, the DNA topoisomerase inhibitor is doxorubicin.

In a further preferred embodiment according to the invention, the DNA cross-linkers are selected from the group consisting of cisplatin, mitomycin C, busulfan, cyclophosphamide and diaziquone.

The drugs with complex effects are in a preferred embodiment according to the invention selected from the group consisting of actinomycin D, bleomycin and temozolomide.

The epigenetic modifiers are in a preferred embodiment according to the invention selected from the group consisting of 5-aza-2'-deoxycytidine, sodium butyrate, trichostatin A, MS-275, SAHA, LBH589, phenylbutyric acid, valproic acid, curcumin, C646 and BRD4770.

In a further preferred embodiment according to the invention, the inhibitors of telomerase activity are selected from the group consisting of SYUIQ-5, BMVC4, pyridostatin, compound 115405, perylene and indole derivatives, harmine, BIBR1532 and azidothymidine.

The inhibitors of cyclin-dependent kinases are in another preferred embodiment according to the invention selected from the group consisting of palbociclib, roscovitine and ribociclib.

Preferably, the activators of p53 are selected from the group consisting of nutlin 3a and FL118.

The activators of protein kinase C are preferably selected from the group consisting of TPA/PMA, PEP005 and PEP008.

The reactive oxygen species inducers are preferably selected from the group consisting of hydrogen peroxide, tert-butyl hydroperoxide, phenyl-2-pyridyl ketoxime, phenylaminonaphthoquinones and paraquat.

In a preferred embodiment according to the invention, the virus induced diseases are diseases induced by human papillomavirus or molluscum contagiosum virus. Human papillomavirus (HPV) is a DNA virus from the papillomavirus family. Over 170 types have been described. About 90% of HPV infections cause no symptoms and resolve spontaneously within two years. However, in some cases, an HPV infection persists and results in either warts or precancerous lesions. These lesions, depending on the site affected, increase the risk of cancer of the cervix, vulva, vagina, penis, anus, mouth, or throat. Nearly all cervical cancer is due to HPV; two types, HPV16 and HPV18, account for 70% of cases. Between 60% and 90% of the other cancers listed above are also linked to HPV. HPV6 and HPV11 are common causes of genital warts and laryngeal papillomatosis. The molluscum contagiosum virus (MCV) is a poxvirus spread either by direct contact, including sexual activity, or via contaminated objects such as towels. The condition can also be spread to other areas of the body by the person themselves. Risk factors include a weak immune system, atopic dermatitis, and crowded living conditions. Following one infection, it is possible to get reinfected. Diagnosis is typically based on the appearance. Molluscum contagiosum (MC), sometimes called water warts, is a viral infection of the skin, caused by MCV, results in small, raised, pink lesions with a dimple in the center. They may occasionally be itchy or sore. They may occur singularly or in groups. Any area of the skin may be affected, with abdomen, legs, arms, neck, genital area, and face being most common. Onset of the lesions is around seven weeks after infection.

In a preferred embodiment of the invention, the virus induced disease is a viral infection of the skin. More preferably, the viral infection of the skin is one or more viral skin warts.

In a more preferred embodiment according to the invention, the one or more compound, wherein the compound is an inducer of stress-induced premature senescence, are for use in the treatment and/or prevention of viral skin warts. In a most preferred embodiment of the invention, doxorubicin is used for the treatment and/or prevention of viral skin warts.

Another aspect of the application provides pharmaceutical compositions which may be prepared by mixing one or more compounds of the invention, pharmaceutically acceptable salts thereof, with pharmaceutically acceptable additives, and pharmaceutically acceptable carriers to treat or prevent virus induced diseases, in particular viral infections of the skin, more particularly skin warts. The composition of the invention may be used to create formulations that would treat virus-induced diseases. Such composition can be in the form of, for example, tablets, capsules, syrup, powder, granules, suspensions, or solutions. The composition according to the invention can be formulated for various routes of administration, for example, by oral administration, by nasal administration, subcutaneous injection, intravenous injection, intramuscular injection, or intraperitoneal injection. The following dosage forms are given by way of example and should not be construed as limiting the present invention.
For oral administration, powders, suspensions, granules, tablets, pills, capsules, and gelcaps are acceptable as solid dosage forms. This can be prepared by mixing one or more compounds of the present invention, with pharmaceutical acceptable salts, and additives such as starch or other additives. Optionally, oral dosage forms can contain other ingredients to aid in administration, such as an active diluent, or lubricants such as magnesium stearate, or preservatives such as parben or sorbic acid, or antioxidants such as ascorbic acid, tocopherol, or cysteine, a disintegrating agent, binders, thickeners, buffers, sweeteners, flavoring agents or perfuming agents. Tablets and pills may be further treated with suitable coating materials known in the art.

Liquid dosage forms for oral administration maybe in the form of pharmaceutically acceptable emulsions, syrups, suspensions or solutions, which may contain an inactive diluent, such as water. Pharmaceutical formulations and medicaments maybe prepared as liquid suspensions or solutions using a sterile liquid, such as, but not limited to, an oil, water, an alcohol, and combination of these.
For nasal administration, the pharmaceutical formulations maybe a spray or aerosol containing an appropriate solvent(s) and optionally other compounds such as, but not limited to, stabilizer, antimicrobial agents, antioxidants, pH modifiers, surfactants, bioavailability modifiers and combination of these. A propeller for an aerosol formulation may include compressed air, nitrogen, carbon dioxide, or a hydrocarbon-based low boiling solvent(s).
Injectable dosage forms generally include aqueous suspensions or oil suspensions which maybe prepared using a suitable dispersant or wetting agent. Injectable forms maybe in solution phase or in the form of a suspension, which is prepared with a solvent or diluent. Acceptable solvents or vehicles include sterilized water, Ringer's solution or isotonic aqueous saline solution. Alternatively, aqueous oil maybe employed as solvents or suspending agents. Typically, the oil or fatty acid is non-volatile, including natural or synthetic oils, fatty acids, mono-, di-, tri-glycerides.
For injection, the pharmaceutical formulation may also be a powder suitable for reconstitution with an appropriate solution as described above. Examples of these include, but not limited to, freeze dried, or spray dried powders, amorphous powders, granules, precipitates or particulates. For injection, the formulation may contain stabilizers, pH modifiers, surfactants, bioavailability modifiers and combination of these.
Specific dosages maybe adjusted depending on conditions of the disease, the age, body weight, general health conditions, sex, and diet of the subject, dose intervals, administration routes, excretion rate, and combination of drugs. Any of the above dosage forms containing effective amounts are well within the scope of the invention.

A therapeutically effective amount of the one or more compounds of the present invention may vary depending upon the route of administration and dosage form. The typical compound or compounds of the invention is a formulation that exhibits a high therapeutic index. The therapeutic index is the dose ration between toxic and therapeutic effects which can be expressed as the ratio between LD50 and ED50. The LD50 is the dose lethal to 50% of the population and the ED50 is the dose therapeutically effective in 50% of the population. The LD50 and ED50 are determined by standard pharmaceutical procedures in animal cell cultures or experimental animals.

Therefore, in one embodiment the present application relates to a pharmaceutical composition for treating virus-induced diseases, in particular viral infections of the skin, more particularly viral skin warts, containing at least one inducer of stress-induced premature senescence as defined above.

In a preferred embodiment according to the invention, the pharmaceutical composition comprises the one or more compound in a concentration of from about 0.01 to about 10% b.w., more preferably about 0.02 to about 5% b.w. and particularly preferred in a concentration from about 0,05 to about 2% b.w.- calculated on the total composition.

In a further embodiment, the present invention relates to a method of treating virus-induced diseases, in particular viral infections of the skin, more particularly viral skin warts, comprising the steps of
a) selecting one or more inducer of stress-induced premature senescence as defined above, and
b) applying said compound to a person in need thereof.

### Examples

### MATERIALS & METHODS

### 2D-LC-MS/MS and proteins identification

For each condition (proliferating and senescent), 3 donors of Normal human Dermal Fibroblast are used. 10 µg of each sample are prepared for Mass spectrometry (MS) analysis. MS analysis of proteome is carried out using 2D-LC-MS/MS system and a TMT6plex label quantification approach. MS/MS analysis is performed using a Q-Exactive™ Hybrid QuadrupoleOrbitrap™ mass spectrometer equipped with a nano-electrospray ion source. LC-MS/MS analysis is performed in 2 technical replicates. Proteins are identified and quantified using Proteome Discoverer search engine (version 2.3.0.523). MS/MS spectra are searched against the UniProtKB database. Search parameters are as follows: Enzyme Name: Trypsin / Max. Missed Cleavage Sites: 3 / Precursor Mass Tolerance: 12ppm / Fragment Masse Tolerance: 0.04Da. The false discovery rate is set to 1 % (p ≤ 0.01). A minimum of 2 unique peptides are required and only proteins found in all samples are retained for quantification. Then, protein abundance is compared across samples by taking the protein abundance ratio senescent/proliferating.

### Gene set enrichment analysis

Gene set enrichment analysis (also functional enrichment analysis) is a method to identify classes of genes or proteins that are over-represented in a large set of genes or proteins, and may have an association with disease phenotypes. The method uses statistical approaches to identify significantly enriched or depleted groups of genes. Gene set enrichment analysis of upregulated proteins (230) is performed using g: Profiler web tool (version e98_eg45_p14_ce5b097, 2/10/2020, h. sapiens). Input is ordered by importance (fold-change, decreasing manner) and correction for multiple testing performed using Benjamini-Hochberg FDR method (threshold 0.05). Results obtained mining GO:BP and KEGG databases. To avoid limited interpretative value of large pathways and statistical power decrease due to small pathways, the size of functional category is set between 5 (min) and 350 (max) genes.

### Results

230 proteins are found upregulated in senescent neonatal human dermal cells (NHDF) and are subjected to a gene set enrichment analysis (GSEA). Viral defense related biological processes were manually selected (see table 1). Also, 53 biological processes involved in immune response & inflammation are identified.

**Table 1: Viral defense-related biological processes enriched in senescent skin fibroblasts. Viral defense mechanisms were manually selected and listed according to their adjusted p-value.**

| **No** | **Name** | **Term ID** | **Adjusted *p*-value** |
|---|---|---|---|
| 1 | Defense response to virus | GO:0051607 | 3.33E-10 |
| 2 | Response to virus | GO:0009615 | 4.24E-08 |
| 3 | Negative regulation of viral process | GO:0048525 | 6.64E-07 |
| 4 | Negative regulation of multi-organism process | GO:0043901 | 2.47063E-06 |
| 5 | Negative regulation of viral life cycle | GO:1903901 | 3.92919E-06 |
| 6 | Negative regulation of viral genome replication | GO:0045071 | 1.19819E-05 |
| 7 | Viral life cycle | GO:0019058 | 6.91743E-05 |
| 8 | Regulation of viral process | GO:0050792 | 7.21356E-05 |
| 9 | Regulation of viral life cycle | GO:1903900 | 8.54942E-05 |
| 10 | Regulation of viral genome replication | GO:0045069 | 9.76198E-05 |
| 11 | Viral genome replication | GO:0019079 | 0.000347587 |
| 12 | Interaction with host | GO:0051701 | 0.000628839 |
| 13 | Regulation by virus of viral protein levels in host cell | GO:0046719 | 0.000648272 |
| 14 | Entry into host | GO:0044409 | 0.000794284 |
| 15 | Entry into host cell | GO:0030260 | 0.000794284 |
| 16 | Positive regulation of defense response to virus by host | GO:0002230 | 0.002322416 |
| 17 | Viral entry into host cell | GO:0046718 | 0.003458454 |
| 18 | Regulation of defense response to virus by host | GO:0050691 | 0.004815527 |
| 19 | Hepatitis C | KEGG:05160 | 0.005780154 |
| 20 | Multi-organism intracellular transport | GO:1902583 | 0.009306718 |
| 21 | Multi-organism cellular localization | GO:1902581 | 0.009306718 |
| 22 | Measles | KEGG:05162 | 0.012528465 |
| 23 | Detection of virus | GO:0009597 | 0.012968918 |
| 24 | Cellular response to dsRNA | GO:0071359 | 0.017272391 |
| 25 | Negative regulation of viral entry into host cell | GO:0046597 | 0.018306373 |
| 26 | Regulation of defense response to virus | GO:0050688 | 0.020738386 |
| 27 | Influenza A | KEGG:05164 | 0.030590211 |
| 28 | Positive regulation of viral genome replication | GO:0045070 | 0.03143238 |
| 29 | Response to exogenous dsRNA | GO:0043330 | 0.033695872 |
| 30 | Response to dsRNA | GO:0043331 | 0.035603171 |
| 31 | Positive regulation of viral life cycle | GO:1903902 | 0.04234752 |
| 32 | Negative regulation of defense response | GO:0031348 | 0.047065767 |
| 33 | Multi-organism localization | GO:1902579 | 0.047493025 |
| 34 | Multi-organism transport | GO:0044766 | 0.047493025 |

Taken together, viral defense mechanisms, immune response and inflammation accounts for -13% of all enriched biological processes in senescent NHDF.

## Claims

1. One or more compound for use in the treatment and/or prevention of virus induced diseases, wherein the one or more compound is an inducer of stress-induced premature senescence.

2. The one or more compound for use according to claim 1, wherein the one or more compound is selected from the group consisting of DNA replication stress inducers, DNA-damaging agents, epigenetic modifiers, inhibitors of telomerase activity, inhibitors of cyclin-dependent kinases, activators of p53, activators of protein kinase C and reactive oxygen species inducers.

3. The one or more compound for use according to claim 2, wherein the DNA replication stress inducers are selected from the group consisting of aphidicolin, hydroxyurea, thymidine, bromodeoxyuridine, difluorodeoxycytidine and cyclopentenyl cytosine.

4. The one or more compound for use according to claim 2, wherein the DNA-damaging agents are selected from the group consisting of DNA topoisomerase inhibitors, DNA cross-linkers and drugs with complex effects.

5. The one or more compound for use according to claim 4, wherein the DNA topoisomerase inhibitors are selected from the group consisting of doxorubicin, etoposide, daunorubicin, mitoxantrone and camptothecin.

6. The one or more compound for use according to claim 4, wherein the DNA cross-linkers are selected from the group consisting of cisplatin, mitomycin C, busulfan, cyclophosphamide and diaziquone.

7. The one or more compound for use according to claim 4, wherein the drugs with complex effects are selected from the group consisting of actinomycin D, bleomycin and temozolomide.

8. The one or more compound for use according to claim 2, wherein the epigenetic modifiers that inhibit DNA enzymes are selected from the group consisting of 5-aza-2'-deoxycytidine, sodium butyrate, trichostatin A, MS-275, SAHA, LBH589, phenylbutyric acid, valproic acid, curcumin, C646 and BRD4770.

9. The one or more compound for use according to claim 2, wherein the inhibitors of telomerase activity are selected from the group consisting of SYUIQ-5, BMVC4, pyridostatin, compound 115405, perylene and indole derivatives, harmine, BIBR1532 and azidothymidine.

10. The one or more compound for use according to claim 2, wherein the inhibitors of cyclin-dependent kinases are selected from the group consisting of palbociclib, roscovitine and ribociclib,

11. The one or more compound for use according to claim 2, wherein the activators of p53 are selected from the group consisting of nutlin 3a and FL118.

12. The one or more compound for use according to claim 2, wherein the activators of protein kinase C are selected from the group consisting of TPA/PMA, PEP005 and PEP008.

13. The one or more compound for use according to claim 2, wherein the reactive oxygen species inducers are selected from the group consisting of hydrogen peroxide, tert-butyl hydroperoxide, phenyl-2-pyridyl ketoxime, phenylaminonaphthoquinones and paraquat.

14. The one or more compound for use according to any of the preceding claims, wherein the virus induced diseases are diseases induced by human papillomaviruses or molluscum contagiosum virus.

15. The one or more compound for use according to any of the preceding claims, wherein the virus induced disease is a viral infection of the skin.
